# EUROPÄISCHE PATENTSCHRIFT

(11) **EP 0 491 127 B1**
(45) Veröffentlichungstag und Bekanntmachung des Hinweises auf die Patenterteilung: **30.07.1997**
(21) Anmeldenummer: 91117448.0
(22) Anmeldetag: 12.10.1991
(51) Int. Cl.: C07C 255/13, C11B 9/00, A61K 7/46

(54) **3-(Cis-3-Hexenyloxy)-propan-nitrile, dessen Herstellung und dessen Verwendung**
3-(Cis-3-hexenyloxy)-propane-nitrile, its preparation and its use
-3-(Cis-3-hexényloxy)-propane-nitrile, sa préparation et son usage

(30) Priorität: 20.11.1990 DE 4037345
(43) Veröffentlichungstag der Anmeldung: 24.06.1992
(73) Patentinhaber: Dragoco Gerberding & Co Aktiengesellschaft, D-37603 Holzminden (DE)
(72) Erfinder: Brunke, Ernst-Joachim, Dr. Dipl.-Chem., W-3450 Holzminden (DE); Fahlbusch, Karl-Georg, Dr.Dipl.-Chem., W-3470 Höxter 1 (DE)
(74) Vertreter: Eikenberg, Kurt-Rudolf, Dr. Dipl.-Chem.

(56) Entgegenhaltungen:
- DE-A- 2 601 825
- DE-A- 2 639 182
- Grur Int. (1986)8/9 550-552
- Riechstoffe, Aromen, Körperflegemittel 2/75, 34-35
- Ullmann's Encyclopedia of Industrial Chemistry 5th Ed., Band A11, S. 145,146,148,152(1990)
- Perfumer & Flavorist, Vol 11 (1986) 27-44
- SÖFW-Journal 121 (9/95) 641-649

## Beschreibung

Gegenstand der Erfindung ist ein neues 3-(Hexenyloxy-propan-nitril) mit der Formel

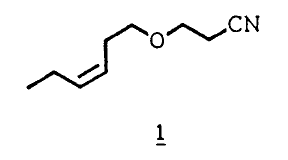

Die Herstellung dieses neuen Oxy-propan-Nitrils erfolgt in an sich bekannter Weise durch Addition eines α,β-ungesättigen Nitrils an cis-3-Hexenol in Gegenwart von Basen als Katalysator. Die neue Verbindung der Formel (1) besitzt außergewöhnliche Geruchseigenschaften und kann daher als Riechstoff sowie Bestandteil von Parfümölen verwendet werden.

In der Riechstoff-Industrie besteht ein ständiger Bedarf an neuen, olfaktorisch originellen, hautverträglichen und stabilen Verbindungen. Diese Anforderungen werden von verschiedenen Substanzklassen zum Teil erfüllt. Von industrieller Bedeutung sind einige aliphatische Nitrile, die sich durch gute Stabilität auszeichnen. Hervorzuheben sind hier Geranylnitril, Citronellylnitril und Tridecen-2-Nitril, die über citrusartige Geruchsnoten bei hoher Intensität und Stabilität verfügen. Derartige Nitrile verfügen allerdings auch über fettige und metallische Nebennoten, die ihrem Einsatz in Parfümölen Grenzen setzen. Über Geruchseigenschaften von Oxy-propan-nitrilen (auch β-Oxynitrile genannt) ist in der Literatur relativ wenig beschrieben worden:
a) Die Deutsche Offenlegungsschrift 26 39 182 beschreibt die Verbindung 3-(10-Undecenyloxy)-propion-nitril als eine Verbindung mit einem schwach-fruchtigen Geruch und einer salicylat-artigen Note mit pudriger Charakteristik.
b) Die Deutsche Offenlegungsschrift 26 01 825 beschreibt Oxy-propannitrile, hergestellt aus Acrylnitril und einerseits gesättigten Fettalkoholen und andererseits aus Terpenalkoholen, wobei die resultierenden Äthernitrile Gerüche besitzen sollen, die an die zugrundeliegenden Alkohole erinnern.
c) In der Zeitschrift "Riechstoffe, Aromen, Körperpflegemittel" 1975, S. 34 werden die Synthese und die Geruchseigenschaften gewisser β-Oxynitrile beschrieben. Acrylnitril wird hier an gesättigte, aliphatische Alkohole, an Terpenalkohole bzw. an cyclische und aromatische Alkohole addiert. Für die entstandenen β-Oxynitrile werden Geruchsbeschreibungen angegeben.

Unter den bisher bekannten 3-Oxynitrilen oder Nitrilen existieren keine Verbindungen mit natürlicher Blättergrün-Note. Es ist daher neu und überraschend, daß die hier beschriebene Verbindung der Formel (1) eine natürlich wirkende Grünnote besitzt, die an den Duft von Veilchenblättern erinnert.

Für die Additionsreaktion von Acrylnitril an Alkohole können verschiedene basische Katalysatoren eingesetzt werden (Organic Reactions, Bd V; 1952, S. 89 Cyanoethylation und dort zitierte Literatur). So verläuft die Reaktion mit Natrium, Natrium-methanolat, Natriumhydroxid oder auch mit der quartären Ammonium-Base Triton B (Benzyltrimethylammonium-hydroxid) bei Temperaturen von 10° - 50° C in guten Ausbeuten.

Die Verbindung der Formel (1) gehört zu einer Gruppe neuer 3-(Hexenyloxy)-propan-nitrile der allgemeinen Formel **A** 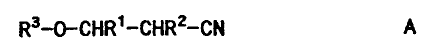 verzweigten in der R³ einen Hexenylrest und R¹ bzw. R² fakultativ ein Wasserstoffatom hat oder eine Methylgruppe sind. Die Verbindungen der Formel **A**, hierbei die erfindungsgemäße Verbindung 1 und die weiteren Verbindungen 2 -9, können durch analoge Reaktion von Acrylnitril, bzw. Methyl-substituiertem Acrylnitril mit unverzweigten Hexenolen hergestellt werden.

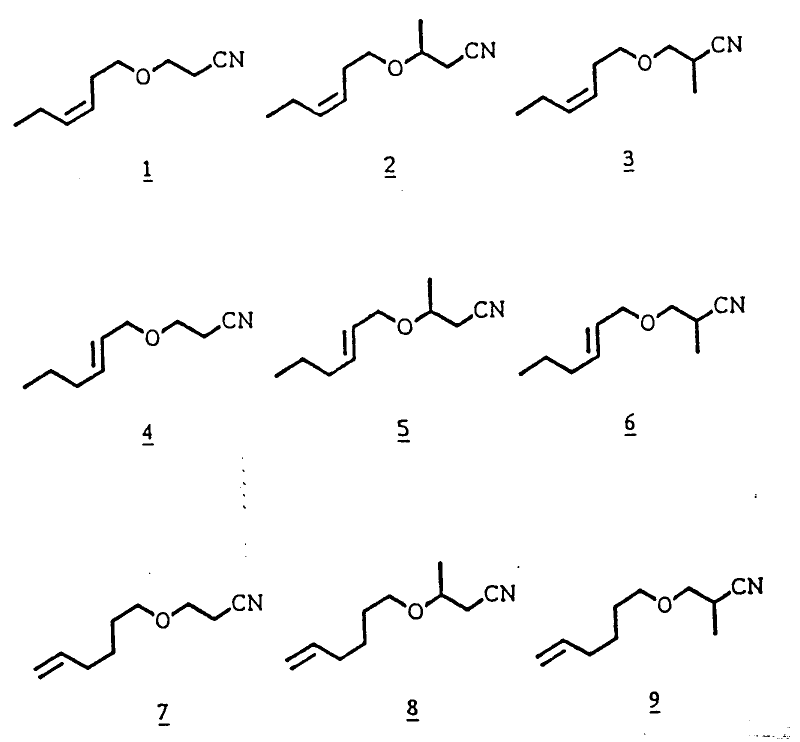

Die neuen Verbindungen der Formel **A** verfügen über bemerkenswerte Geruchseigenschaften, die überwiegend in eine natürlich-grüne Duftrichtung gehen und sich durch erhebliche Duftstärke auszeichnen; die Geruchscharakteristik der Oxynitrile **A** ist deutlich verschieden von derjenigen der jeweiligen Ausgangsalkohole. Neben diesen starken grünen Noten besitzt beispielsweise das erfindungsgemäße 3-(cis-Hex-3'-enyl-oxy)-propan-nitril (**1**) auch fruchtige und blumige Aspekte mit einer starken Ausstrahlung in der Kopfnote. Überraschenderweise besitzen die Verbindungen der Formel **A**, die im Gegensatz zu den Ausgangsalkoholen sowohl im basischen als auch im sauren Bereich stabil sind, eine wesentlich stärkere Haftfestigkeit auf Fasern als die zugrundeliegenden Alkohole.

Diese physikalisch-chemischen Eigenschaften und die starken frisch-grünen Geruchsnoten sind wertvolle Eigenschaften für die Parfümerie. Die Verbindungen der Formel **A** eignen sich besonders gut zum Verbessern, Verstärken oder Verändern des Charakters von Parfümölen.
Beispiel 1 : Darstellung der Alkenyl-oxy-propan-nitrile 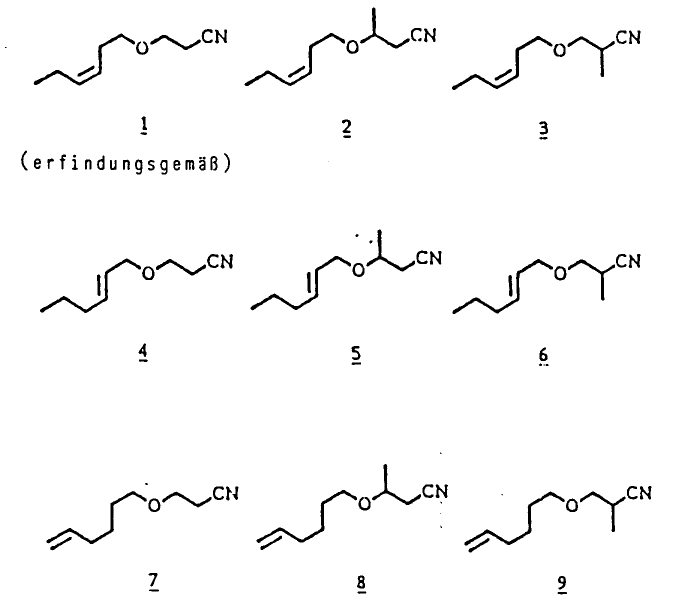 In einem 500 ml Rührwerk (Rückflußkühler, Thermometer, Rührmotor, Tropftrichter) wurden
1 mol *des ungesättigten, unverzweigten Alkohols*
und
0,01 mol des Phasentransferkatalysators *Triton B* vorgelegt.
Hierzu tropfte man innerhalb von 4 bis 6 Std.
1,1-1,2 mol des *ungesättigten Nitriles* bei folgenden Temperaturen zu:
0 - 10 °C bei *Acrylnitril*
15 - 25 °C bei *Crotonnitril*
30 - 40 °C bei *Methacrylnitril*
Nach etwa 15 h Rühren bei Raumtemperatur wurde mit etwa der gleichen Meng*e Diethylether*, sodann mit ca. 100 ml *Wasser* und 5 - 10 ml 10%ige *Schwefelsäure* versetzt. Das Gemisch wurde jeweils intensiv geschüttelt. Die organische Phase wurde jeweils mit gesättigter Kochsalzlösung gewaschen, über Natriumsulfat getrocknet und unter vermindertem Druck eingeengt. Das Rohprodukt wurde jeweils über eine 20 cm Glasfüllkörperkolonne destilliert. Man erhielt die in der nachfolgenden Tabelle angegebenen Ausbeuten.

| Nr | R³-O-CHR¹⁻CHR²⁻CN | n²⁰ D | D²⁰ 4 | Siedepunkt [°C / mm] | Ausbeute [%] | Geruchsbeschreibung |
|---|---|---|---|---|---|---|
| 1 | R¹=H | 1.4456 | 0.9074 | 86 / 2 | 80 | stark, grün, etw. fruchtig blumig (Veilchenblätter) |
| | R²=H | | | | | |
| | R³=cis-3-hexenyl | | | | | |
| 2 | R¹=CH3 | 1.4439 | 0.8930 | 93 / 3 | 72 | stark, grün, blumig,aldehydisch etas Gurke |
| | R²=H | | | | | |
| | R³=cis-3-hexenyl | | | | | |
| 3 | R¹=H | 1.4413 | 0.8875 | 100 / 2.5 | 66 | grün, blumig, fruchtig (Banane, Melone) |
| | R²=CH3 | | | | | |
| | R³=cis-3-hexenyl | | | | | |
| 4 | R¹=H | 1.4441 | 0.9014 | 93 / 1 | 87 | stark, süß, fruchtig (Birne, Apfel), krautig, blumig |
| | R²=H | | | | | |
| | R³=trans-2-hexenyl | | | | | |
| 5 | R¹=CH3 | 1.4434 | 0.8917 | 98 / 0.6 | 76 | grün, etw. krautig-chemisch |
| | R²=H | | | | | |
| | R³=trans-2-hexenyl | | | | | |
| 6 | R¹=H | 1.4406 | 0.8860 | 49 / 0.5 | 56 | grün, pilzig, etwas metallisch |
| | R²=CH3 | | | | | |
| | R³=trans-2-hexenyl | | | | | |
| 7 | R¹=H | 1.4404 | 0.9011 | 96 / 3 | 77 | stark, grün, fruchtig (Apfel), aldehydisch |
| | R²=H | | | | | |
| | R³=5-hexenyl | | | | | |
| 8 | R¹=CH3 | 1.4404 | 0.8901 | 70 / 0.5 | 55 | grün, etwas metallisch |
| | R²=H | | | | | |
| | R³=5-hexenyl | | | | | |
| 9 | R¹=H | 1.4376 | 0.8844 | 65 / 0.5 | 58 | stark, grün, fruchtig, etwas süß |
| | R²=CH3 | | | | | |
| | R³=5-hexenyl | | | | | |

Beispiel 2 : Spektroskopische Daten von 3-(cis-Hex-3'-enyl-oxy)-propan-nitril (1)
Bruttoformel: C ₉ H ₁₅ N O ( 153,23 )
IR (Film) : 2254 (CN), 1654 (C=C), 1113 (C-O-C) cm⁻¹.
MS : m/e = 153 (2%, M⁺), 138 (2%), 124 (4%), 110 (7%), 97 (5%), 84 (19%), 82 (38%), 69 (19%), 67 (65%), 54 (100%), 41 (63%).
Beispiel 3 : Spektroskopische Daten von 3-(cis-Hex-3'-enyl-oxy)-butan-nitril (2)
Bruttoformel: C₁₀H₁₇NO (167,25)
IR (Film) : 252 (CN), 1652 (C=C), 1104 (C-O-C) cm⁻¹.
¹H-NMR (CDCl₃, 300 MHz): δ = 5.45 (m; 1H, H-olefin.), 5.35 (m; 1H, H-olefin.), 3.72 (sex.; J=6.1 HZ; 2H, CH₂ ), 3,47(m, 2H, CH₂), 2.50 (d; J=5.7 Hz; 2H, CH₂⁾, 2.31 (q, J=7.7 Hz; 2H, CH₂), 2.06 (p, J=7.7 Hz; 2H, CH₂),1.29 (d; J=6.1 Hz; 3H, CH₃), 0.96 (t, J=7.5 Hz; 3H, CH₃).
¹³C-NMR (CDCl₃, 75 MHz) : δ = 133.8 (C-olefin.; d), 124.7 (C-olefin.; d), 117.5 (CN; s), 71.2 (C-1'; t), 68.9 (C-3; d), 28.0 (C-2; t), 25.0 (C-2'; t), 20.7 (C-5'; t), 19.7 (C-4; q), 14.2 (C-6'; q).
MS : m/e = 167 (1%, M⁺), 152 (1%), 138 (1%), 124 (2%), 111 (2%), 98 (10%), 82 (40%), 68 (100%), 67 (37%), 55 (12%), 41 (15%).
Beispiel 4 : Spektroskopische Daten von 3-(cis-Hex-3'-enyl-oxy)-2-methyl-propan-nitril (3)
Bruttoformel: C₁₀H₁₇NO (167.25)
IR (Film) : 2245 (CN), 1653 (C=C), 1120 (C-O-C) cm⁻¹.
¹H-NMR (CDCl)₃, 300 MHz): δ = 5.45 (m, 1H, H-olefin.), 5.35 (m, 1H, H-olefin.), 3.49 (t, J=6.9 Hz; 2H, CH₂), 3,49-3.57 (m; 2H, CH₂) , 2.85 (sex., J=6.1 Hz; 1H, CH), 2.33 (q, J=7.0 Hz; 2H, CH₂), 2.06 (p, J=6.1 Hz; 2H, CH₂), 1.32 (d, J=7.1 Hz; 3H, CH₃), 0.97 (t, J=7.5 Hz; 3H, CH₃).
¹³C-NMR (CDCl₃, 75 MHz) : δ = 133.9 (C-olefin.; d), 124.6 (C-olefin.; d), 121.4 (CN; s), 71.5 (C'-1; t), 71.2 (C-3; t), 27.7 (C-2'; t), 26.6 (C-2; d), 20.7 (C-5'; t),
Beispiel 5 : Stabilitätstests mit der Verbindung **1**

Die erfindungsgemäße Verbindung **1** wurde in marktübliche Grundmassen in ebenfalls für Parfümöle marktüblichen Dosierung eingearbeitet. Die geruchliche Beurteilung erfolgte einen Tag nach der Einarbeitung bzw. nach einem Monat oder drei Monaten Lagerung bei Raumtemperatur und einem Monat oder drei Monaten Lagerung bei 40 °C. Die Beurteilung "sehr gut" (SG) bedeutet, daß keine geruchliche Veränderung festzustellen war. Die Beurteilung "gut" (G) bedeutet eine leichte, jedoch akzeptable Veränderung des Geruchs, während die Beurteilung "nicht gut" (NG) eine deutliche Geruchsveränderung bedeutet. Bei den Geruchsbeurteilungen wurde mit einem bei -18 °C aufbewahrten Standardmuster (in der jeweiligen Grundmasse) verglichen.

Bei Bestrahlung mit synthetischem Tageslicht trat bei weißer Seife eine geringfügige Verfärbung auf, die vergleichbar ist mit derjenigen von Amylzimtaldehyd oder Methyljonon, bei gleicher Grundmasse und gleicher Dosierung.

| Grundmasse | Dosierung[%] | Standard | 1 Monat Raumtemp | 1 Monat 40°C | 3 Monate Raumtemp. | 3 Monate 40°C |
|---|---|---|---|---|---|---|
| saurer Reiniger (pH 1,8) (H₃PO₄) | 0,2 | SG | SG | SG | SG | G |
| saurer Reiniger (pH 1,8) (HCHOOH) | 0,3 | SG | SG | G | G | G |
| alkal.Reiniger (pH 10,5) | 0,4 | SG | SG | G | SG | G |
| Seife, weiß | 1,0 | SG | SG | SG | G | G |
| Waschmittel ohne TAED | 0,2 | SG | G | G | G | G |
| Waschmittel mit TAED | 0,2 | SG | SG | G | SG | G |
| Weichspüler | 0,2 | SG | SG | SG | SG | G |
| Schaumbad | 1,5 | SG | SG | SG | SG | G |
| Shampoo | 0,5 | SG | G | G | G | G |
| Aerosol/Antiperspirant | 0,4 | SG | G | G | G | G |
| Deo-Spray | 0,3 | SG | SG | SG | G | G |
| Tages-Creme ö/e-Emuls. | 0,3 | SG | G | G | G | G (-) |
| Creme w/ö-Emuls. | 0,3 | SG | G | G (-) | G | G (-) |

Beispiel 6: Parfümkomposition grün-holzigen Typs

| | **A** | **B** |
|---|---|---|
| 3-(cis-Hex-3'-enyl-oxy)-propannitril (**1**) | - | 1 |
| Bergamotte-Öl | 10 | 10 |
| Beifuss-Öl | 1,5 | 1,5 |
| Dihydromyrcenol | 5 | 5 |
| Lavandin-Öl, Typ grosso | 2 | 2 |
| β-Jonon | 1 | 1 |
| Timberol ^{R} (DRAGOCO) | 10 | 10 |
| Iso-E-Super ^{R} (IFF) | 15 | 15 |
| Patchouli-Öl | 3 | 3 |
| Lignofix ^{R} (Acetylcedrene, DRAGOCO) | 10 | 10 |
| Hedione ^{R} (Firmenich) | 5 | 5 |
| Galaxolide ^{R} 50 (IFF) | 18 | 18 |
| Lyral ^{R} (IFF) | 8 | 8 |
| Sandranol ^{R} (DRAGOCO) | 3 | 3 |
| Benzylsalicylat | 5 | 5 |
| Dipropylenglycol | 3,5 | 2,5 |
| | 100,0 | 100,0 |

Die Mischung **A** besitzt einen ausgewogenen Duft aus grüneragrumiger Ambra-Note mit blumigen (Veilchen) und holzigen Elementen. In der Mischung **B** wird das blumige Element durch die starke grün-blumige Frische der erfindungsgemäßen Verbindung **1** entfaltet und ausgewogen. Der Charakter der Kopfnote wird durch die Verbindung **1** verstärkt.
Beispiel 7 : Parfümkomposition blumigen Typs

| | **A** | **B** |
|---|---|---|
| 3-(cis-Hex-3'-enyl-oxy)-propannitril (**1**) | - | 2,4 |
| Benzyl-acetat | 7 | 7 |
| Di-(Methylbutyl)-carbinyl-acetat | 8,6 | 8,6 |
| Methyl-Jonon, gamma | 24 | 24 |
| α-Jonon | 6 | 6 |
| Hydroxinal extra (DRAGOCO) | 16 | 16 |
| Hedione ^{R} (Firmenich) | 10 | 10 |
| Isodamascone ^{R} (DRAGOCO) | 2 | 2 |
| Exaltolide ^{R} (Firmenich) | 8 | 8 |
| Benzylsalicylat | 16 | 16 |
| Dipropylenglycol | 2,4 | - |
| | 100,0 | 100,0 |

Die Mischung **A** besitzt klassisch-blumige Elemente. Im Accord **B** wird durch die Zugabe der erfindungsgemäßen Verbindung **1** ein stark-grüner veilchenartiger Charakter hervorgerufen. Hier stellt das Oxonitril **1** einen wesentlichen Bestandteil dieses Blumen-Accords dar.

## Patentansprüche (Patentansprüche für folgende(n) Vertragsstaat(en): AT, BE, CH, DE, DK, FR, GB, GR, IE, IT, LI, LU, MC, NL, PT, SE)

1. 3-(cis-3-Hexenyloxy)-propan-nitril.

2. Verwendung von 3-(cis-3-Hexenyloxy)-propan-nitril als Riechstoff bzw. als Bestandteil von Parfümkompositionen.

## Patentansprüche (Patentansprüche für folgende(n) Vertragsstaat(en): ES)

1. Verfahren zur Herstellung eines neuen 3-Hexenyloxy-propan-nitrils mit der Formel 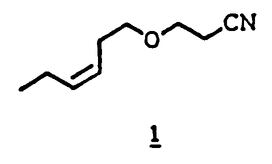 gekennzeichnet durch die Addition von Acrylnitril an cis-3-Hexenol in Gegenwart von Basen als Katalysator.

2. Verwendung von 3-(cis-3-Hexenyloxy)-propan-nitril als Riechstoff bzw. als Bestandteil von Parfümkompositionen.

## Claims (Claims for the following Contracting State(s): AT, BE, CH, DE, DK, FR, GB, GR, IE, IT, LI, LU, MC, NL, PT, SE)

1. 3-(cis-3-hexenyloxy)-propane-nitrile.

2. Use of 3-(cis-3-hexenyloxy)-propane-nitrile as odorant or as component of perfume compositions, respectively.

## Claims (Claims for the following Contracting State(s): ES)

1. A process for the preparation of a new 3-hexenyloxy-propane-nitrile of formula 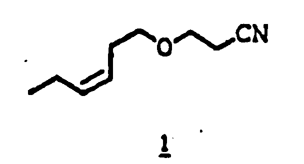 characterized by the addition of acrylonitrile to cis-3-hexenol in the presence of a base a catalyst.

2. Use of 3-(cis-3-hexenyloxy)-propane-nitrile as odorant or as component of perfume compositions, respectively.

## Revendications (Revendications pour l'(les) Etat(s) contractant(s) suivant(s): AT, BE, CH, DE, DK, FR, GB, GR, IE, IT, LI, LU, MC, IL, PT, SE)

1. 3-(cis-3-hexenyloxy)-propanenitrile.

2. Utilisation de 3-(cis-3-hexenyloxy)-propanenitrile comme matière odorante ou comme composent de compositions de parfum.

## Revendications (Revendications pour l'(les) Etat(s) contractant(s) suivant(s): ES)

1. Procédé pour la fabrication d'un nouveau 3-(cis-3-hexenyloxy)-propanenitrile avec la formule 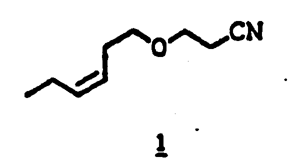 carcatérisé par l'addition d'acrylenitrile au cis-3-hexenol en présence de bases comme catalysateur.

2. Utilisation de 3-(cis-3-hexenyloxy)-propanenitrile comme matière odorante ou comme composant de compositions de parfum.
